# EUROPEAN PATENT APPLICATION

(11) **EP 1 542 011 A1**
(43) Date of publication of application: **15.06.2005**
(21) Application number: 03797662.8
(22) Date of filing: 18.09.2003
(51) Int. Cl.: G01N 33/53, G01N 33/543, G01N 33/531

(54) **METHOD OF MEASURING COLLAGEN**

(30) Priority: 18.09.2002 JP 2002271200
(71) Applicant: Kudoh, Norio, Chiyoda-ku, Tokyo 102-0075 (JP)
(72) Inventor: MATSUMOTO, Kazuko, Tokyo 155-0032 (JP)
(74) Representative: Sternagel, Fleischer, Godemeyer & Partner
(86) International application number: PCT/JP2003/011900
(87) International publication number: WO 2004/027423

(57) **Abstract**

It is an object of the present invention to provide a novel method for measuring collagen, which is capable of measuring a small amount of collagen contained in a small amount of sample, when only a small amount of sample, such as in the case of mouse urine, is obtained. The present invention provides a method for measuring collagen by immunoanalysis using an anti-collagen antibody which is **characterized in that** a test sample is treated with a reducing agent.

## Description

### TECHNICAL FIELD

The present invention relates to a method for measuring collagen; more specifically, it relates to a method for measuring collagen in a small amount of sample with high sensitivity, and to a kit for measuring collagen.

### BACKGROUND ART

Type IV collagen is one of the principal components of renal extracellular matrix, which is released from the glomerular and tubular basement membrane in renal tissues (T. Iijima et al., J. Clini. Lab. Anal., 1998, 12, 378-382). Urinary excretion of the type IV collagen increase in accordance with the progression of various renal diseases as a result of the increase of type IV collagen in the diseased kidney.

Recent clinical trials to find a better indicator for diabetic nephropathy have demonstrated that the urinary levels of type IV collagen are considered to reflect precisely the rate of matrix turnover (H. Okonogi et al., Clin. Nephrol., 2001, 55, 357-364; H. Makino et al., Res. Commmun. Mol. Pathol. Pharmacol., 1995, 88, 215-223; and M. P. Cohen et al., Diabetes Care, 2001, 24, 914-918). Moreover, type IV collagen is also considered to be a better indicator than urinary albumin in kidney disease such as diabetic nephropathy (T. Iijima et al., J. Clini. Lab. Anal., 1998, 12, 378-382; M. P. Cohen et al., Diabetes Care, 2001, 24, 914-918; N. Banu et al., Diabetes Res. Clin. Pract., 1995, 29, 57-67; and N. Kotajima et al., J. Diabetes Complications, 2000, 14, 13-17). Although various ELISA and radioimmunoassay (RIA) systems to measure human type IV collagen have been used in clinical investigations (J. Risteli et al., Anal. Biochem., 1981, 113, 372-378; and K. Obata et al., Clin. Clim. Acta., 1989, 181, 293-304), there have been no practical method for measuring mouse type IV collagen that is capable of measuring a small amount of collagen in a very small amount of urine to be analyzed.

Type IV collagen is a large polymer with unique structural features including self-aggregating properties (R. Timpl et al., Eur. J. Biochem., 1981, 120, 203-211; P. D. Yurchenco et al., Biochem., 1984, 23, 1839-1850; P. D. Yurchenco et al., J. Histochem. Cytochem., 1986, 34, 93-102; C. Johansson et al., J. Biol. Chem., 1992, 267, 24533-24537; B. Siebold et al., Eur. J. Biochem., 1987, 168, 569-575; P. D. Yurchenco et al., J. Cell Biol., 1987, 105, 2559-2568; and R. Dölz et al., Eur. J. Biochem., 1988, 178, 357-366). Each type IV collagen molecule is composed of three α-chains that can be divided into three domains: 7S, a minor collagenous domain in the N-terminal region; a major collagenous domain in the middle region; and NC1, a noncollagenous globular domain in the C-terminal region). The trimers of α-chains are organized into triple helices in the 7S and the major collagenous domains, but in the NC1 domain each chain is folded in a globular structure, stabilized by intrachain disulfide bonds. Using the 7S and NC1 domains as cross-linking domains, four type IV collagen molecules are connected in the 7S domain, and two molecules in the NC1 domain, giving rise to a large polygonal network of collagen type IV molecules (R. Timpl et al., Eur. J. Biochem., 1981, 120, 203-211). An important structural characteristic, on which we have particularly focused, is that disulfide bridges serve as a major bonds, used in 88% of intramolecular bridges to form the polygonal macromolecular network by bonding each unit (B. Sieboled et al., Eur. J. Biochem., 1988, 176, 617-624).

### DISCLOSURE OF THE INVENTION

It is an object of the present invention to provide a novel method for measuring collagen, which is capable of measuring a small amount of collagen contained in a small amount of sample, when only a small amount of sample, such as in the case of mouse urine, is obtained.

In order to reflect accurately early renal damage in experimental mouse models, the present inventors have attempted to establish a highly sensitive time-resolved fluoroimmunoassay (TR-FIA) to measure type IV collagen levels in small amounts of moue urine, which exists in only an extremely small amount in a very small amount of analyte. Based on the type IV collagen structural features mentioned above, the present inventors first pre-treated samples with dithiothreitol to reduce the number of intermolecular disulfide bridges. This pretreatment with dithiothreitol enabled us to measure mouse urinary type IV collagen more accurately without requiring a large amount of urine volumes. The usefulness of the method of the present invention was confirmed by comparing the urinary levels of type IV collagen both in KK/Ta mouse (a diabetic strain spontaneously exhibiting type 2 diabetes; H. Ikeda, Diabetes Res. Clin. Pract., 1994, 24 (Suppl.), 313-316) and in BALB/c mouse used as a control mouse that did not have diabetes, using both the method of the present invention and common competitive ELISA not using DTT. The present invention has been completed based on these findings.

That is to say, the present invention provides a method for measuring collagen by immunoanalysis using an anti-collagen antibody which is characterized in that a test sample is treated with a reducing agent.

Preferably, the immunoanalysis is fluoroimmunoassay using an anti-collagen antibody. More preferably, it is sandwich-type time-resolved fluoroimmunoassay (TR-FIA).

The collagen is preferably type IV collagen.

The reducing agent is preferably dithiothreitol.

The test sample is preferably urine, and more preferably the urine of an experimental animal.

The concentration of the reducing agent in a test sample is preferably between 0.1 mM and 10 mM, more preferably between 0.1 mM and 5 mM, and further more preferably between 0.5 mM and 5 mM.

The method for measuring collagen of the present invention preferably comprises the following steps:
(1) a step of treating a test sample with a reducing agent;
(2) a step of allowing the test sample treated in (1) above to come into contact with a solid phase carrier on which an anti-collagen immobilized antibody is immobilized;
(3) a step of allowing an anti-collagen labeled antibody having a labeling substance to come into contact with the above-described solid phase carrier; and
(4) a step of detecting or measuring collagen which was bound to the anti-collagen immobilized antibody by using the above-described labeling substance.

In another aspect, the present invention provides a kit for measuring collagen, which comprises at least a reducing agent and an anti-collagen antibody.

### BRIEF DESCRIPTION OF THE DRAWINGS

Figure 1 shows a calibration curves of mouse type IV collagen by TR-FIA (A) and competitive ELISA (B). Each point represents the mean ± SD from four experiments.
Figure 2 shows a correlation between concentrations of mouse urine containing type IV collagen and fluorescence signal intensity of TR-FIA. Concentration before (shown by open squares and circles) and after addition of DTT (shown by closed squares and circles) were measured using KK/Ta and BALB/c urine samples. Each point represents the mean ± SD from three experiments.
Figure 3 shows the effect of DTT concentration on urine and a standard sample. Urine samples from KK/Ta mice and a standard solution were incubated with equal volumes of 0, 2 and 5 mM DTT.
Figure 4 shows a comparison of mouse urinary type IV collagen levels between KK/Ta (n = 6) and BALB/c (n = 6) mice at the age of 20 weeks. The measurements were performed by TR-FIA (A) and commercial competitive ELISA (B).
   * KK/Ta significantly higher than BALB/c (p< 0.01).
Figure 5 shows the mRNA expression of type IV collagen (α1 chain) isolated from whole kidney of KK/Ta and BALB/c mouse.

### BEST MODE FOR CARRYING OUT THE INVENTION

The embodiments of the present invention will be described below.

The present invention provides a highly sensitive immunoassay method for measuring a small amount of collagen such as type IV collagen in mouse urine. In the present invention, on the basis of the structural features of a type IV collagen molecule, a test sample is pretreated with dithiothreitol (DTT). The detection limit is 2.4 ng/ml in conventional competitive ELISA that does not involve DTT treatment. In contrast, the method of the present invention enables the measurement of 27 pg/ml type IV collagen contained in 5 µl of a urine sample.

The amounts of type IV collagen in urine derived from 12 samples from 2 types of mouse strains (KK/Ta and BALB/c) were measured by the method of the present invention. As a result, it was found that the amount of type IV collagen in the urine of a diabetic mouse clearly differs from that of a non-diabetic mouse. When compared with a case where no DTT treatment is carried out, the method of the present invention enables the measurement of collagen contained in a very small amount of sample. Thus, the method of the present invention is suitable for the measurement of type IV collagen in urine that is a limited amount of analyte, and in particular, in that of a mouse.

The method of the present invention for measuring collagen by immunoanalysis using an anti-collagen antibody is characterized in that a test sample is treated with a reducing agent.

The test sample used in the present invention is not particularly limited, as long as it is used to measure an amount of collagen. Examples of such a test sample may include any given body fluids of mammals such as a human, a rat, or a mouse, including urine, blood, serum, blood plasma, tissue fluid, saliva, and sweat. The method for measuring collagen of the present invention can be used to measure a small amount of collagen contained in a very small amount of sample with high sensitivity. Thus, urine of experimental animals such as a mouse, which is generally collected at a small amount and which is difficult to collect in a large amount, can be used as a test sample.

The type of collagen measured by the present invention is not particularly limited. Collagen contained in Vertebrata is classified into categories ranging from type I to type XXV. In the present invention, any of the aforementioned types may be measured. Of these types, type IV collagen can particularly preferably be measured in the present invention.

The reducing agent used in the present invention is not particularly limited, as long as it is capable of reducing a disulfide bond in collagen. Examples of such a reducing agent may include dithiothreitol (DTT) and β-mercaptoethanol. Particularly preferably, dithiothreitol (DTT) can be used.

In the method of the present invention, a test sample is previously treated with the aforementioned reducing agent. In the treatment with the reducing agent, the concentration of the reducing agent in the test sample is preferably between 0.1 mM and 10 mM, more preferably between 0.1 mM and 5 mM, and further more preferably between 0.5 mM and 5 mM. For example, it can be set at approximately 2 mM. If the concentration of the reducing agent is lower than 0.1 mM, the action of the reducing agent to reduce a disulfide bond is not sufficiently exerted. On the other hand, if the concentration of the reducing agent is higher than 10 mM, it is likely to affect the three-dimensional structure of epitope of collagen molecule. Thus, it is not preferable.

The immunoanalysis of the present invention uses an anti-collagen antibody.

The anti-collagen antibody used in the present invention may be either a monoclonal antibody or a polyclonal antibody. Such an antibody can be produced by a common method for producing an antibody. Moreover, as such an antibody, not only IgG, but also an antibody fragment (for example, a F(ab')₂ fragment, a Fab' fragment, etc.) may be used.

For example, a polyclonal anti-collagen antibody can be produced by immunizing a mammal with collagen as an antigen, collecting the blood from the mammal, and then separating and purifying an antibody from the collected blood. Examples of a mammal to be immunized may include a mouse, a hamster, a Guinea pig, a chicken, a rat, a rabbit, a dog, a goat, a sheep, and a bovine. The method of administering an antigen is known to persons skilled in the art. The administration route is not particularly limited. An administration route can be selected as appropriate from subcutaneous administration, intracutaneous administration, intraperitoneal administration, intravenous administration, and intramuscular administration. In addition, an adjuvant can be used as necessary. The immunized mammal is bred for an appropriate period of time, and then a small amount of serum is sampled from the ear vein or the like of the mammal. Thereafter, an antibody titer thereof is measured. When the antibody titer increases, an antigen may be administered thereto for booster according to the circumstances. One or two months after the final administration, the blood is collected from the immunized animal by ordinary methods. The collected blood is then separated and purified by ordinary methods, such as centrifugation, precipitation using ammonium sulfate or polyethylene glycol, chromatography such as gel filtration chromatography, ion exchange chromatography, or affinity chromatography, so as to obtain a polyclonal anti-collagen antibody in the form of polyclonal antiserum.

A monoclonal antibody can be obtained by producing a hybridoma by the cell fusion between antibody-generating cells and myeloma cells. Examples of antibody-generating cells used herein may include spleen cells, lymph node cells, and B lymphocytes, collected from immunized animals. For example, spleen cells used as antibody-generating cells are collected from an immunized animal, and these cells are then fused with myeloma cells by a known method (G. Kohler et al., Nature, 256 495(1975)), so as to produce a hybridoma. Examples of a myeloma cell line used in cell fusion may include mouse P3X63Ag8 line, mouse P3U1 line, and mouse Sp2/0 line. When cell fusion is carried out, fusion promoters such as polyethylene glycol or Sendai virus can be used, and when hybridomas are selected after cell fusion, hypoxanthine aminopterin thymidine (HAT) medium can be used according to conventional methods. Hybridomas obtained by cell fusion are then cloned by limiting dilution or the like. Thereafter, the obtained hybridomas are screened by enzyme immunoassay using collagen, so as to obtain a cell line that generates a monoclonal antibody specifically recognizing the desired collagen. In order to produce a monoclonal antibody of interest from a hybridoma, the hybridoma may be cultured by the common cell culture method or ascites formation method, and then, the monoclonal antibody may be purified from the culture supernatant or ascites. The monoclonal antibody can be purified from the culture supernatant or ascites by conventional methods. For example, methods such as ammonium sulfate fractionation, gel filtration, ion exchange chromatography, or affinity chromatography can appropriately be applied in combination.

Examples of the method for measuring collagen of the present invention include fluorescence immunoassay, enzyme immunoassay, radioimmunoassay, and luminescence immunoassay. For example, an anti-collagen antibody is allowed to bind to an insoluble carrier, so as to prepare an antibody-bound insoluble carrier. Using this carrier, sandwich-type immunoassay can be carried out. For example, when such sandwich-type immunoanalysis is carried out by applying the method of the present invention, the present method comprises the following steps:
(1) a step of treating a test sample with a reducing agent;
(2) a step of allowing the test sample treated in (1) above to come into contact with a solid phase carrier on which an anti-collagen immobilized antibody is immobilized;
(3) a step of allowing an anti-collagen labeled antibody having a labeling substance to come into contact with the above-described solid phase carrier; and
(4) a step of detecting or measuring collagen which was bound to the anti-collagen immobilized antibody by using the above-described labeling substance.

An anti-collagen antibody used as a labeled antibody is labeled with a labeling substance, and preferably with a nonradioactive labeling substance. Examples of such nonradioactive labeling used herein may include enzyme labeling, fluorescent labeling, and luminescent labeling. For enzyme labeling, alkaline phosphatase (ALP), β-D-galactosidase, horseradish peroxidase, and other enzymes can be used as labeling enzymes. In order to detect these enzymes, their enzyme substrates are used. These substrates are 4-methyl umbelliferyl phosphate (4-MUP), a combination of hydrogen peroxide and 3,3',5,5'-tetramethylbentidine, and 2-nitrophenyl-β-D-galactoside. These enzyme substrates change (for example, cleave) due to the action of enzymes and thereby develop color, and thus, such color can be detected.

Based on the measurement principles of devices used, immunoassay is classified as follows. In the present invention, any of these methods may be used.
(A) Chemical luminescent immunoassay devices
   1. CLEIA (Chemical Luminescent Enzyme Immuno Assay)
      Chemical luminescence is developed with enzymes.
   2. CLIA (Chemical Luminescent Immuno Assay)
      Chemical luminescence is developed with catalysts.
   3. ECLIA (Electro Chemical Luminescent Immuno Assay)

   Chemical luminescence is developed by electric change.
(B) Radioimmunoassay devices
   1. Radio Immuno Assay (RIA)
      This is a competitive method.
   2. Immuno Radio Metric Assay (IRMA)
(C) Enzyme immunoassay devices
   1. Enzyme Immuno Assay (EIA)
   2. ELISA method (Enzyme Linked Immuno Sorbent Assay)
(D) Fluorescence or fluorescence polarization immunoassay devices
   1. Fluorescent Enzyme Immuno Assay (FEIA)
   2. Fluorescent Polarization Immuno Assay (FPIA)
   3. Time-Resolved Fluorometry (TR-F)
(E) Latex agglutination immunoassay device
(F) Nepherometric immunoassay device
(G) Immunosensors
   1. Electrochemical immunosensor
   2. Optical immunosensor
   3. Thermal immunosensor
   4. Piezo acoustic immunosensor

The immunoanalysis of the present invention is preferably fluoroimmunoassay, and particularly preferably sandwich-type time-resolved fluoroimmunoassay (TR-FIA).

Among trivalent rare earth ions, it has been known that when samarium (Sm), europium (Eu), terbium (Tb), or dysprosium (Dy) ions form a complex with a specific ligand, these ions emit strong fluorescence that is specific for metal ions. The fluorescence of these complexes has the following characteristics:
(1) The complexes have an extremely long fluorescence lifetime. A rare earth fluorescent complex, and in particular, a complex of europium and terbium, has a fluorescence lifetime of several hundreds of microseconds or longer.
(2) The complexes have an extremely large Stokes shift. In the majority of cases, these complexes are excited by adsorption of ultraviolet light, and emit visible light of 500 nm or more.
(3) The half value width of the fluorescence emission peak is between approximately 10 and 20 nm, and thus it is very sharp. Utilizing such fluorescence properties, time-resolved fluoroimmunoassay using a rare earth fluorescent complex as a labeling substance has been developed. In the time-resolved fluoroimmunoassay, various types of background fluorescence with short lifetimes can be eliminated, and thus, highly sensitive measurement is made possible.

Moreover, the present invention provides a kit for measuring collagen. The kit of the present invention comprises at least a reducing agent and an anti-collagen antibody. As the anti-collagen antibody, two types of antibodies, namely an immobilized antibody and a labeled antibody, may be contained. The kit of the present invention may further comprise a reagent for detecting the aforementioned labeling substance.

The present invention will be more specifically described in the following examples. However, the examples are not intended to limit the scope of the present invention.

### EXAMPLES

### (A) Materials and methods

### (1) Experimental animals

The animal study was conducted in accordance with the guidelines of the animal facility of Juntendo University. For this study, male of two inbred mice (KK/Ta and BALB/c) were purchased at the age of 4 weeks from CLEA Japan, Inc. (Tokyo, Japan). From 6 weeks onward, mice were individually housed in plastic cages with free access to food (rodent pellet chow CE-2; 342.2 kcal/100 g, containing 4.4% crude fat) and water throughout the experimental period. Only male mice were used in this study. All mice were kept in the same room under conventional conditions with a regular light cycle of 12 hours light and 12 hours darkness, and the temperature controlled at 24 ± 1°C. When the mice were 20 weeks old, a glucose tolerance test was carried out. As a result, it was confirmed that KK/Ta mice were fully diabetic. Urine samples were collected repeatedly for 10 days because a large urine volume was required for the comparison. Glucose tolerance was assessed using the intraperitoneal glucose tolerance test (IPGTT) for mice. IPGTT was performed by injecting glucose (2g/kg in 20% solution) intraperitoneally in overnight-fasted mice. Glucose levels in the blood obtained from the retro-orbital sinus were measured using Glutest E (Kyoto Daiichi Kagaku, Kyoto, Japan) at 0 (fasting blood sugar levels) and 120 minute after intraperitoneal glucose injection. Body weight was also measured at the same age.

### (2) Antibodies and antigens

Rabbit polyclonal anti-mouse type IV collagen antibody was purchased from NOVOTEC (St. Martin La Garenne, France). Biotinylated goat polyclonal anti-human type IV collagen antibody, which crossreacts wth mouse type IV collage, was obtained from Abcam Limited (Cambridge, U. K.). Type IV collagen from Engelbreth-Holm-Swarm transplantable mouse tumor (Sigma-Aldrich Coro., St. Louis, MO, U.S.A.) was used as a standard antigen.

### (3) Labeling of streptoavidin with BHHCT

Streptoavidin was obtained from Chemicon International Inc. (Temecula, CA, U.S.A.). The streptoavidin-BSA conjugate (SA-BSA) was prepared as reported previously (J. Yuan, G. Wang, et al., Anal. Biochem., 1997, 254, 283-287). The SA-BSA conjugate was labeled with a chelate compound, BHHCT as described previously (J. Yuan, G. Wang, et al., Anal. Biochem., 1997,254,283-287). The solution was diluted by adding 0.05 M Tris-HCl buffer (pH 7.8) containing 1.0 x 10⁻⁷ M EuCl₃, 0.2% BSA, 0.1% NaN₃, and 0.9% NaCl (dilution buffer), and was reacted at 56°C for 2 hours prior to used in immunoassay.

### (4) Measurement of mouse urinary type IV collagen using the time-resolved fluoroimmunoassay (TR-FIA)

Urine samples were first incubated with equal volumes of 2 mM dithiothreitol (Invitrogen corp, CA, U.S.A.) at 37°C for 30 minutes. Standard type IV collagen solution (2,000 ng/ml) was also incubated with 2 mM DTT in the same way. After dilution of samples with dilution buffer, 50 µl of urine or standard solution was added into the wells of a microtiter plate for fluorometry (Nalge Nunc International, N.Y., U.S.A), which had been coated with rabbit anti-mouse type IV collagen antibody (50 µl of 1:200 diluted antibody/well) . After incubation at 5°C overnight, the wells were washed 3 times with a Tris-HCl buffer containing 0.05% Tween 20, and 50 µl of the biotinylated anti-type IV collagen antibody (1 : 300 diluted) was added. After incubation at 5°C overnight, 50 µl of the BHHCT-Eu³⁺ labeled SA-BSA conjugate (SA-BSA-BHHCT-Eu³⁺) solution was added, and incubated at 37°C for 1 hour. Finally, the wells were washed 3 times with Tris-HCl buffer (pH 9.1) containing 0.05% Tween 20, and the plate was subjected to solid-phase fluorometric measurement. Time-resolved fluorometric measurement was performed using Arvo SX multilabel counter (PerkinElmer Life Sciences, Boston, MA, U.S.A.), with excitation at 340 nm, delay time of 0.2 ms, and window time of 0.4 ms. Fluorescence intensity was measured at 615 nm.

### (5) Measurement of mouse urinary type IV collagen using competitive ELISA

Urinary type IV collagen was measured by the competitive enzyme-linked immunosorbent assay method (ELISA) (collagen IV M kit, Exocell, Philadelphia, PA, U.S.A.). The assay was performed according to the directions included with the kit, and 240 µl of urine samples was used.

### (6) Creatinine assay

Urinary creatinine was measured using a commercial kit for urine samples (Creatinine Companion, Exocell, Philadelphia, PA, U.S.A.).

### (7) Northern blot analysis for mRNA of type IV collagen

Probes used for Northern blot analysis were prepared as reported previously (E. P. Peten et al., Am. J. Physiol., 1992, 263, 951-957). Total RNA isolation from the whole kidneys of KK/Ta and BALB/c mice at the age of 20 weeks was performed using Trizol (Invitrogen corp., Carlsbad, CA, U.S.A.). RNA (10 µg) was electrophoresed on 1.5% agarose and transferred to the nylon membrane. The membrane was prehybridized for 4 hours, and then hybridized with ³²P-labeled cDNA probes for the α1 chain of mouse type IV collagen and GAPDH overnight. After washing, the membrane was exposed to an image plate (Fuji Photo Film Co., Ltd., Tokyo, Japan). The intensity of bands on the image plate was quantified by densitometric scanning model of BAStation 2500 (Fuji Photo Film Co., Ltd., Tokyo, Japan). Finally, adjustment of intensity of α1 chain bands was performed at the GAPDH band intensities.

### (8) Statistical analysis

Data were expressed as mean ± SD. Statistical significance in between two different mice strains was determined by the Mann-Whitney U test. Regression-correlation analysis in between ELISA and TR-FIA was done using Spearman's test. P values of less than 0.05 were regarded as significant.

### (B) Results

### (1) Comparison of detection limits between competitive ELISA and TR-FIA system

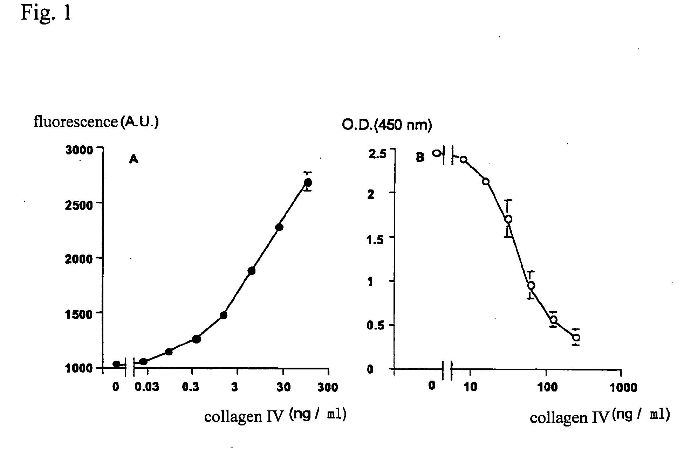

The calibration curves of mouse type IV collagen by competitive ELISA and by TR-FIA are shown in Figure 1. The detection limit of TR-FIA defined using 3 x SD of background was 27 pg/ml by the method reported previously (J. Yuan, G. Wang, et al., Anal. Biochem., 1997, 254, 283-287). The mean coefficient of variation that was simultaneously measured was 4.2% (0.4% to 7.4% at 8 different concentrations). The detection limit of TR-FIA (27 pg/ml) was approximately 1/100 lower than that of competitive ELISA. To obtain recoveries of standard antigens to urine specimens, 1:5 diluted human urine was used as a diluent. Five different concentrations of standard mouse type IV collagen (2.5, 10, 40, 160, and 320 ng/ml) in the diluted human urine was assayed. Analytical recovery of mouse type IV collagen added to human urine samples was more than 95% at each concentration.

### (2) Analysis of diluted mouse urine samples

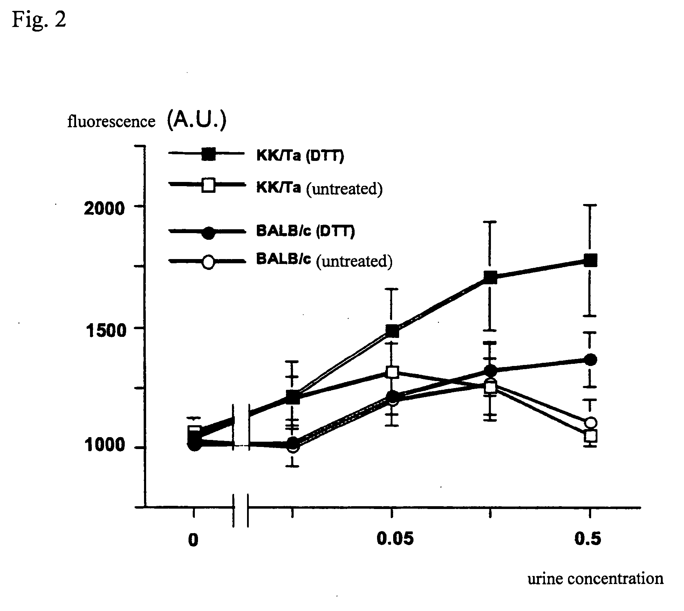

Using urine samples from diabetic KK/Ta and non-diabetic BALB/c mice at the age of 20 weeks, correlation between the concentration of urinary mouse type IV collagen and fluorescence intensity was investigated (Figure 2). The urine samples of each strain (n = 3) were diluted with dilution buffer. When the sample were assayed without pretreatment, fluorescence intensity decreased from dilution 1 : 20 to 1 : 2 (shown by open squares). This trend was more significant in KK/Ta mice than in BALB/c mice. A low recovery rate of antigen in KK/Ta mice may be caused by large molecular size collagen containing many disulfide bridges, which suggests interference in the immune reactions through non-specific adhesion of macromolecules to the wells. Therefore, mouse urine samples were treated with DTT before assay.

### (3) Optimal condition for urinary type IV collagen measurement with DTT pretreatment

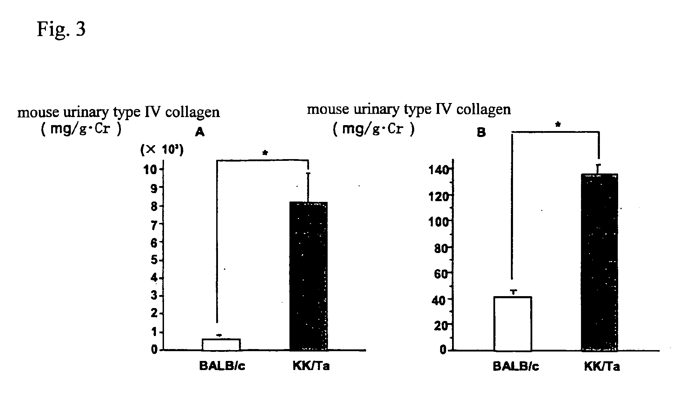

In order to establish optimal conditions for measurement, the effect of DTT concentration in the pretreatment of urine samples was examined (Figure 3A). DTT concentration was varied (1, 2, and 5 mM), and the result is shown in Figure 3. Standard antigen was also treated in the same manner (Figure 3B). In both assays, standard solution was hardly affected at a concentration of 2 mM DTT, the best assay of type IV collagen in urine sample was achieved at 2 mM. By pretreatment with DTT at a concentration of 2 mM before dilution, a correlation between urinary type IV collagen concentration and fluorescence intensity was observed in KK/Ta mice (shown by closed squares in Figure 3B).

To check cross-reactivity with other type collagens such as mouse type I and II collagens, both type collagens were also treated with 2 mM DTT. Cross-reactions with these type collagens were not observed, confirming that pretreatment with DTT does not provide crossing properties to other collagen molecules such as type I or II, and that the specificity of type IV collagen is maintained.

### (4) Glucose tolerance and body weight of KK/Ta and BALB/c mice

As shown in Table 1, KK/Ta mice at the age of 20 weeks showed fasting hyperglycemia and impaired glucose tolerance as well as obesity, compared with the findings in the normal control BALB/c mice at the age of 20 weeks (p < 0.01).

**Table 1:**

| Experimental animal data | | |
|---|---|---|
| | BALB/c | KK/Ta |
| Fasting blood glucose level (mg/dl) | 76.4 ± 9.5 | 110.2 ± 25.3* |
| Blood glucose level at 120 minutes after intraperitoneal glucose injection (mg/dl) | 85.6 ± 13.3 | 434.4 ± 49.8* |
| Body weight (g) | 31.0 ± 0.8 | 42.6 + 2.4* |

| | | |
|---|---|---|
| * The values in KK/Ta are significantly higher than those in BALB/c mice (p< 0.05). | | |

### (5) Measurement of concentration of mouse urinary type IV collagen in two mice strains using TR-FIA and conventional competitive immunoassay

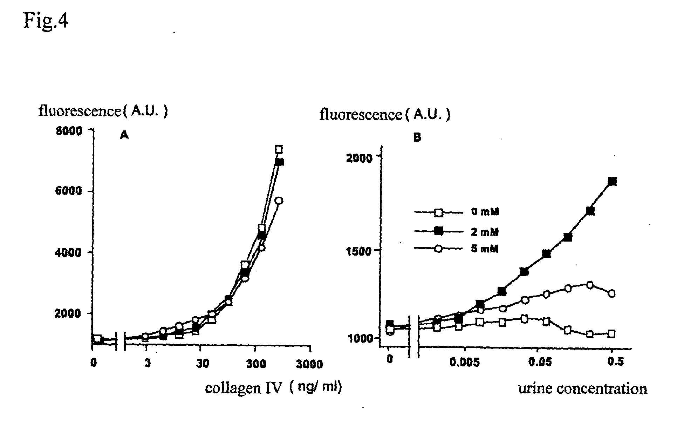

To investigate the usefulness of TR-FIA, mouse type IV collagen was measured at the same time by competitive ELISA using a commercial kit. As shown in Figure 4, concentration of urinary type IV collagen, in diabetic KK/Ta and non-diabetic BALB/c mice at the age of 20 weeks was measured by both TR-FIA and competitive ELISA. In each immunoassay, a higher concentration was observed in KK/Ta mice than in BALB/c mice (p < 0.01), but the range of the concentration in TR-FIA was much higher than that in the competitive ELISA. Furthermore, in TR-FIA the difference of the values between KK/Ta and BALB/c mice was greater than in competitive ELISA. A positive correlation was shown in between these assays (correlation coefficient = 0.607, p value = 0.034).

### (6) Northern blot analysis

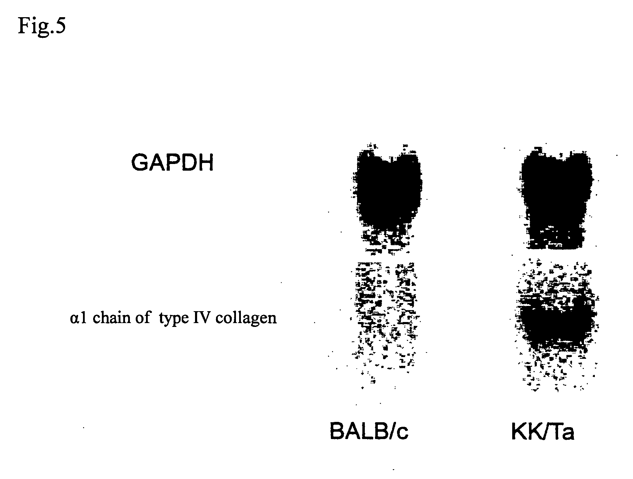

As shown in Figure 5, mRNA expression of type IV collagen increased more significantly in KK/Ta mice than in BALB/c mice. Densitometric scanning showed that the mRNA expression of the α1 chain was 11.1 times higher in KK/Ta mouse than in BALB/c mouse.

### (C) Discussion

Several studies have reported increased production of type IV collagen in diseased kidneys (M. S. Razzaque et al., J. Pathol., 1994, 174, 131-138; and T. A. Jacot et al., Lab. Invest., 1996, 75, 781-799). In addition to these reports, various clinical studies regarding the amount of urinary type IV collagen have been conducted. The results of these clinical studies suggest that an increase in urinary type IV collagen is a better indicator of early renal dysfunction. However, there has been no highly sensitive assay method for very small urine samples, such as those obtained from rodents. Currently used conventional ELISA requires a large sample volume (240 µl), but mouse urine, generally a very small quantity in each excretion, and thus, is difficult to collect. However, TR-FIA using europium chelate compound (BHHCT-Eu³⁺) as a label was found to be applicable to measurement of urinary type IV collagen in extremely small samples.

Type IV collagen is a large molecule having several structural features. According to gel chromatographic analysis, type IV collagen in a urine samples has the maximum peak at 340 kDa (H. Makino et al., Res. Commmun. Mol. Pathol. Pharmacol., 1995, 88, 215-223). As mentioned above, this large molecule consists of polymer units, each with many disulfide bridges as major bonds. In immunoassays, it is generally said that the complexity increases with the molecular size to be measured. In the assay without DTT pretreatment, fluorescent intensity at high concentrations of urine samples was not proportionate to the amount of type IV collagen, in spite of a large amount of collagen excreted in KK/Ta mouse urine. This lack of correlation, appeared to be caused by interference of the antigen-antibody reaction, sterically hindered by a macromolecular network of self-aggregating properties.

In antigen-antibody reactions, antibody is generally considered to recognize only the peptide on the surface of an antigen (G. S. Page et al., J. Virol., 1988, 62, 1781-1794). It was reported that type IV collagen was efficiently extracted from an Engelbreth-Holm-Swarm mouse tumor using 2 mM DTT and 2 mM guanidine (H. K. Kleinman et al., Biochemistry, 1982, 21, 6188-6193). In the present invention, a large number of disulfide bridges are reduced with DTT, and the size of a molecule of urinary type IV collagen is thereby decreased, so that an antibody could sufficiently recognize an epitope on the surface of a collagen molecule. As a result, pretreatment by addition of DTT to urine sample made is possible to reproduce a positive correlation between the concentration of urinary type IV collagen and signal strength on TR-FIA. Disulfide bridges are generally considered to serve as a part of intermolecular and intramolecular bonds, which are associated with confirmation of type IV collagen molecule. Addition of more than 2 mM DTT to the standard solution did not give a better result, which suggests that the excessive pretreatment might affect intramolecular disulfide bonds, and thus that it might change the conformation of epitopes in the antigen.

Although TR-FIA using the pretreatment with DTT and competitive ELISA using a commercial kit showed the same trend with the concentration of urinary type IV collagen in KK/Ta mice higher than that in BALB/c mice, several clear differences were observed in these immunoassays.

The range of the concentration in TR-FIA was nearly 100 times higher than that in competitive ELISA. This may be attributed to the following differences: assay methods, antibodies used and addition of DTT. Addition of DTT might contribute to further enhancement of differences between KK/Ta and BALB/c mice in TR-FIA, more than in competitive ELISA. Similar to the results in TR-FIA, Northern blot analysis also showed that mRNA expression of type IV collagen increased much more in KK/Ta mouse than in BALB/c mouse. This large difference at the expression level clearly supports the results of TR-FIA analysis. TR-FIA requires only 5 µl of urine sample, while competitive ELISA required as much as 240 µl. Since pretreatment with DTT was performed in TR-FIA, it required only a small amount of sample.

### INDUSTRIAL APPLICABILITY

The method of the present invention enables the measurement of a very small amount of collagen contained in a very small amount of sample. In addition, the method of the present invention can clearly express a difference in the amount of collagen among various mouse strains, without conducting a cross-reaction with another type of collagen. It is expected that early renal dysfunction in an experimental model mouse can be evaluated by the method of the present invention more sensitively than by the conventional assay using a protein such as albumin as an indicator.

## Claims

1. A method for measuring collagen by immunoanalysis using an anti-collagen antibody which is **characterized in that** a test sample is treated with a reducing agent.

2. The method for measuring collagen according to claim 1 wherein the immunoanalysis is fluoroimmunoassay using an anti-collagen antibody.

3. The method for measuring collagen according to claim 1 or 2 which is sandwich-type time-resolved fluoroimmunoassay (TR-FIA).

4. The method for measuring collagen according to any of claims 1 to 3 wherein the collagen is type IV collagen.

5. The method for measuring collagen according to any of claims 1 to 4 wherein the reducing agent is dithiothreitol.

6. The method for measuring collagen according to any of claims 1 to 5 wherein the test sample is urine.

7. The method for measuring collagen according to any of claims 1 to 6 wherein the test sample is urine of an experimental animal.

8. The method for measuring collagen according to any of claims 1 to 7 wherein the concentration of the reducing agent in a test sample is between 0.1 mM and 10 mM.

9. The method for measuring collagen according to claim 8 wherein the concentration of the reducing agent in a test sample is between 0.1 mM and 5 mM.

10. The method for measuring collagen according to claim 8 wherein the concentration of the reducing agent in a test sample is 0.5 mM and 5 mM.

11. The method for measuring collagen according to any of claims 1 to 10 which comprises the following steps:
(1) a step of treating a test sample with a reducing agent;
(2) a step of allowing the test sample treated in (1) above to come into contact with a solid phase carrier on which an anti-collagen immobilized antibody is immobilized;
(3) a step of allowing an anti-collagen labeled antibody having a labeling substance to come into contact with the above-described solid phase carrier; and
(4) a step of detecting or measuring collagen which was bound to the anti-collagen immobilized antibody by using the above-described labeling substance.

12. A kit for measuring collagen, which comprises at least a reducing agent and an anti-collagen antibody.
